# EUROPEAN PATENT APPLICATION

(11) **EP 4 563 010 A1**
(43) Date of publication of application: **04.06.2025**
(21) Application number: 23847037.1
(22) Date of filing: 28.07.2023
(51) Int. Cl.: A23L 33/10, A23L 2/385, A23L 29/00, A61K 8/9789, A61K 8/36, A61Q 19/08, A61Q 90/00, C12N 1/20, C12N 1/18

(54) **FERMENTED APPLE COMPOSITION**

(30) Priority: 29.07.2022 KR 20220094863
(71) Applicant: CJ CheilJedang Corporation, Seoul 04560 (KR)
(72) Inventor: JUNG, Hee Kyoung, Seoul 04560 (KR); JEON, Kyung Chan, Seoul 04560 (KR); HONG, Na Youn, Seoul 04560 (KR); PARK, Yeeun, Seoul 04560 (KR); KIM, Sunhee, Seoul 04560 (KR); LEE, Eunyong, Seoul 04560 (KR)
(74) Representative: Meissner Bolte Partnerschaft mbB
(86) International application number: PCT/KR2023/011025
(87) International publication number: WO 2024/025381

(57) **Abstract**

The present disclosure relates to a novel fermented apple composition and use thereof.

## Description

### [Technical Field]

The present disclosure relates to a novel fermented apple composition and use thereof.

### [Background Art]

Photoaging is a phenomenon in which collagens in the dermal layer are degraded by UV-generated reactive oxygen species (ROS) in skin cells. When ROS generated by UV stimulation penetrates into cells and induces expression of matrix metalloproteinase-1 (MMP-1), which is an enzyme that degrades collagen, the overexpressed MMP-1 degrades collagen in the dermal layer of the skin, resulting in loss of skin elasticity and aging. Therefore, in order to prevent or inhibit photoaging, it is necessary to develop a substance capable of effectively inhibiting MMP-1 expression.

In addition, the intestine, which is the main place where substances introduced from the outside come into contact, plays an important role in protecting our body from various external stimuli. Intestinal defense is primarily based on the structural features and intrinsic motility of the intestinal tract, and the defense mechanism acts secondarily by cellular and humoral immunity related to the permeability of intestinal epithelial cells.

The tight junctions that exist between cells affect the permeability of intestinal epithelial cells. When tight junctions loosen due to stimuli or damage in the intestine, harmful molecules (pathogens, toxins, antigens, *etc..)* excessively permeate the intestinal epithelium, causing endotoxemia, disrupting the mucosal immune system and inducing an inflammatory response, leading to bowel diseases or systemic diseases with inflammation.

Inflammatory bowel disease (IBD), which shows a recent increasing trend of incidence among bowel diseases, is a disease caused by neutrophils as a mediator. These neutrophils are attracted by interleukin-8 (IL-8), which is increased in inflamed mucosa (Non-Patent Document 0001). IL-8 is one of inflammatory cytokines, involved in tumorigenesis and angiogenesis in addition to attracting neutrophils, and plays an important role in various pathological conditions such as chronic inflammation and cancer (Non-Patent Document 0002). For example, various types of cells, including macrophages and tumor cells, secrete IL-8 as an inflammatory response induced by lipopolysaccharide (LPS) stimulation.

Meanwhile, apples are fruits of the apple tree, which is a perennial plant belonging to the genus *Malus,* the family *Rosaceae,* and they have high contents of sugar, vitamin A, vitamin C, and inorganic salts. Vitamin C, which is contained in large amounts in apples, has an excellent effect of regenerating damaged skin cells by strengthening the bond between keratin and collagen in the skin. In addition, an apple extract is known to have effects of soothing and moisturizing the skin, and apples and apple juice are known to have skin exfoliation effects by containing malic acid, tartaric acid, and amylase enzyme. In addition, apples have a high content of dietary fiber such as pectin, organic acid, sugar, vitamins, and inorganic salts. In particular, pectin, which is a water-soluble dietary fiber contained in large amounts in apples, is effective for constipation by helping intestinal movement, and organic acids are well known to help digestion and absorption by promoting secretion of gastric juice.

Currently, various food and cosmetic compositions using apples have been developed. However, as there is an increasing domestic and foreign demand for compositions capable of improving skin conditions by preventing or inhibiting photoaging, the development of novel compositions with better effects of preventing or inhibiting photoaging is required.

### [Prior Art Documents]

### [Patent Document]

(Patent Document 1) Korean Laid-Open Publication No. 10-2022-0025679 [Non-Patent Documents]
(Non-Patent Document 1) M C Grimm et al., Gut 1996, pages 90-98.
(Non-Patent Document 2) M Naofimi, Am J Physiol Lung Cell Mol Physiol 284(4), Apr 2003, pages L566-577.

### [Disclosure]

### [Technical Problem]

The present disclosure provides a fermented apple composition and use thereof.

### [Technical Solution]

An object of the present disclosure is to provide a fermented apple composition including 30 g/L to 100 g/L of acetic acid, and any one or more acids selected from the group consisting of succinic acid, lactic acid, formic acid, and gallic acid.

Another object of the present disclosure is to provide a food including the fermented apple composition of the present disclosure.

Still another object of the present disclosure is to provide a health functional food including the fermented apple composition of the present disclosure.

Still another object of the present disclosure is to provide a cosmetic composition including the fermented apple composition of the present disclosure

### [Advantageous Effects]

The novel fermented apple composition of the present disclosure has an effect of reducing or inhibiting UV-induced collagenase expression and the expression of inflammatory cytokines according to the composition and content of specific organic acids

### [Brief Description of the Drawings]

FIG. 1 shows results of examining expression levels of matrix metalloproteinase-1 (MMP-1) by treating UVB-irradiated HaCaT cells with different concentrations of a fermented apple composition or an apple concentrate. * indicates p<0.05, and ** indicates p<0.01.
FIG. 2 shows results of examining expression levels of human interleukin-8 (hIL-8) by concentration by treating HT-29 cells with different concentrations of a fermented apple composition or an apple concentrate together with lipopolysaccharide (LPS). ***p < 0.001 (vs NO group), ###p < 0.001, #p < 0.05 (vs LPS group).

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a fermented apple composition including 30 g/L to 100 g/L of acetic acid, and any one or more acids selected from the group consisting of succinic acid, lactic acid, formic acid, and gallic acid.

As used herein, the term "apple concentrate" means a liquid obtained by concentrating apples, and those prepared or those commercially available may be used without limitation. For example, the apple concentrate may be a 100% apple concentrate, but is not limited thereto. Further, the apple concentrate may have a sugar content of 60 brix or more, based on a refractometer, but is not limited thereto. In addition, the apple concentrate may have an acidity of 6% or less, but is not limited thereto.

As used herein, the term "fermented apple composition" refers to a fermentation product resulting from fermentation of the apple concentrate.

As used herein, the term "fermentation" commonly refers to a phenomenon or process in which microorganisms convert and accumulate metabolites by decomposing organic matter using their own enzymes.

As used herein, the term "fermentation product" may be comprehensively interpreted as including all of a material produced by fermentation of the present disclosure, a filtrate thereof, a dilution thereof, a concentrate thereof, a crude product thereof, a purified product thereof, *etc.,* but is not limited thereto

The fermented apple composition of the present disclosure may be fermented by inoculating an *Acetobacter* sp. strain into an apple concentrate.

The *Acetobacter* sp. strain may be *Acetobacter aceti* and/or *Acetobacter pasteurianus.*

The fermentation by inoculating the *Acetobacter* sp. strain may be performed at a temperature range consisting of one lower limit selected from 20°C or higher, 25°C or higher, 30°C or higher, 35°C or higher, and 40°C or higher, and/or one upper limit selected from 50°C or lower, 45°C or lower, 40°C or lower, 35°C or lower, 30°C or lower, 25°C or lower, and 20°C or lower. In addition, the fermentation by inoculating the *Acetobacter* sp. strain may be performed for a period of time in a range consisting of one lower limit selected from 2 days or more, 3 days or more, 4 days or more, 5 days or more, 6 days or more, and 7 days or more, and/or one upper limit selected from 10 days or less, 9 days or less, 8 days or less, 7 days or less, 6 days or less, 5 days or less, 4 days or less, 3 days or less, and 2 days or less.

In another embodiment, the fermented apple composition of the present disclosure may be fermented by inoculating a *Saccharomyces cerevisiae* strain into an apple concentrate prior to fermentation by inoculating the *Acetobacter* sp. strain.

The fermentation by inoculating the *Saccharomyces cerevisiae* strain may be performed at a temperature range consisting of one lower limit selected from 20°C or higher, 25°C or higher, 30°C or higher, 35°C or higher, and 40°C or higher, and/or one upper limit selected from 50°C or lower, 45°C or lower, 40°C or lower, 35°C or lower, 30°C or lower, 25°C or lower, and 20°C or lower. In addition, the fermentation by inoculating the *Saccharomyces cerevisiae* strain may be performed for a period of time in a range consisting of one lower limit selected from 2 days or more, 3 days or more, 4 days or more, 5 days or more, 6 days or more, and 7 days or more, and/or one upper limit selected from 10 days or less, 9 days or less, 8 days or less, 7 days or less, 6 days or less, 5 days or less, 4 days or less, 3 days or less, and 2 days or less.

The fermented apple composition of the present disclosure may be ripened after inoculating and fermenting with the *Acetobacter* sp. strain.

The ripening may be performed by allowing the fermented apple composition to stand at room temperature. The ripening may be performed at a temperature range consisting of one lower limit selected from 1°C or higher, 3°C or higher, 5°C or higher, 10°C or higher, 15°C or higher, 20°C or higher, 25°C or higher, and 30°C or higher, and/or one upper limit selected from 35°C or lower, 30°C or lower, 25°C or lower, 20°C or lower, 15°C or lower, 10°C or lower, 5°C or lower, 3°C or lower, and 1°C or lower. In addition, the ripening may be performed for a period of time in a range consisting of one lower limit selected from 2 days or more, 3 days or more, 4 days or more, 5 days or more, 6 days or more, and 7 days or more, and/or one upper limit selected from 10 days or less, 9 days or less, 8 days or less, 7 days or less, 6 days or less, 5 days or less, 4 days or less, 3 days or less, and 2 days or less.

The fermented apple composition of the present disclosure may include 30 g/L to 100 g/L of acetic acid, and any one or more acids selected from the group consisting of succinic acid, lactic acid, formic acid, and gallic acid.

Specifically, the fermented apple composition of the present disclosure may include acetic acid in a range consisting of one lower limit selected from 40 g/L or more, 50 g/L or more, 60 g/L or more, 70 g/L or more, and 72 g/L or more, and/or one upper limit selected from 100 g/L or less, 90 g/L or less, 80 g/L or less, 75 g/L or less, and 73 g/L or less.

The fermented apple composition of the present disclosure may not include citric acid, or may include citric acid in an amount of 0.001 g/L or less, and specifically may not include citric acid, or may include citric acid in a range consisting of one upper limit selected from 0.0005 g/L or less, 0.0001 g/L or less, 0.00005 g/L or less, 0.00001 g/L or less, and 0.000001 g/L or less.

The fermented apple composition of the present disclosure may include succinic acid at a concentration of 0.1 g/L to 0.8 g/L. Specifically, the fermented apple composition of the present disclosure may include succinic acid in a range consisting of one lower limit selected from 0.2 g/L or more, 0.3 g/L or more, 0.35 g/L or more, 0.37 g/L or more, and 0.4 g/L or more, and/or one upper limit selected from 0.7 g/L or less, 0.6 g/L or less, 0.5 g/L or less, 0.45 g/L or less, and 0.42 g/L or less.

The fermented apple composition of the present disclosure may include lactic acid at a concentration of 0.2 g/L to 1.5 g/L. Specifically, the fermented apple composition of the present disclosure may include lactic acid in a range consisting of one lower limit selected from 0.4 g/L or more, 0.6 g/L or more, 0.7 g/L or more, 0.75 g/L or more, and 0.8 g/L or more, and/or one upper limit selected from 1.3 g/L or less, 1.1 g/L or less, 1.0 g/L or less, 0.9 g/L or less, and 0.85 g/L or less.

The fermented apple composition of the present disclosure may include formic acid at a concentration of 0.01 g/L to 0.05 g/L. Specifically, the fermented apple composition of the present disclosure may include formic acid in a range consisting of one lower limit selected from 0.012 g/L or more, 0.014 g/L or more, 0.016 g/L or more, 0.018 g/L or more, and 0.02 g/L or more, and/or one upper limit selected from 0.04 g/L or less, 0.05 g/L or less, 0.02 g/L or less, 0.027 g/L or less, and 0.025 g/L or less.

The fermented apple composition of the present disclosure may include gallic acid at a concentration of 0.2 g/L to 0.6 g/L. Specifically, the fermented apple composition of the present disclosure may include gallic acid in a range consisting of one lower limit selected from 0.21 g/L or more, 0.22 g/L or more, 0.23 g/L or more, 0.24 g/L or more, and 0.25 g/L or more, and/or one upper limit selected from 0.5 g/L or less, 0.4 g/L or less, 0.37 g/L or less, 0.36 g/L or less, and 0.35 g/L or less.

In addition, the fermented apple composition of the present disclosure may further include any one or more acids selected from the group consisting of malic acid, chlorogenic acid, caffeic acid, and ferulic acid.

The fermented apple composition of the present disclosure may include malic acid at a concentration of 0.05 g/L to 0.3 g/L. Specifically, the fermented apple composition of the present disclosure may include malic acid in a range consisting of one lower limit selected from 0.07 g/L or more, 0.09 g/L or more, 0.1 g/L or more, 0.12 g/L or more, and 0.13 g/L or more, and/or one upper limit selected from 0.25 g/L or less, 0.22 g/L or less, 0.2 g/L or less, 0.17 g/L or less, and 0.16 g/L or less.

The fermented apple composition of the present disclosure may include chlorogenic acid at a concentration of 1 g/L to 20 g/L. Specifically, the fermented apple composition of the present disclosure may include chlorogenic acid in a range consisting of one lower limit selected from 2 g/L or more, 4 g/L or more, 6 g/L or more, 7 g/L or more, and 8 g/L or more, and/or one upper limit selected from 18 g/L or less, 16 g/L or less, 14 g/L or less, 12 g/L or less, and 10 g/L or less.

The fermented apple composition of the present disclosure may include caffeic acid at a concentration of 0.1 g/L to 0.8 g/L. Specifically, the fermented apple composition of the present disclosure may include caffeic acid in a range consisting of one lower limit selected from 0.2 g/L or more, 0.3 g/L or more, 0.35 g/L or more, 0.37 g/L or more, and 0.4 g/L or more, and/or one upper limit selected from 0.7 g/L or less, 0.6 g/L or less, 0.5 g/L or less, 0.47 g/L or less, and 0.45 g/L or less.

The fermented apple composition of the present disclosure may include ferulic acid at a concentration of 0.05 g/L to 0.3 g/L. Specifically, the fermented apple composition of the present disclosure may include ferulic acid in a range consisting of one lower limit selected from 0.07 g/L or more, 0.09 g/L or more, 0.1 g/L or more, 0.12 g/L or more, and 0.14 g/L or more, and/or one upper limit selected from 0.25 g/L or less, 0.22 g/L or less, 0.2 g/L or less, 0.19 g/L or less, and 0.18 g/L or less.

In the present disclosure, the fermented apple composition of the present disclosure may have an effect of reducing or inhibiting UV-caused collagen degradation.

The inhibiting of collagen degradation may be achieved by, for example, reducing or inhibiting the expression of collagenase.

The collagenase of the present disclosure may be matrix metalloproteinase-1 (MMP-1), MMP-2, or MMP-9, and any collagenase may be included in the scope of the present disclosure, as long as it is known in the art. In one embodiment, the collagenase may be MMP-1.

When UV-irradiated cells are treated with the fermented apple composition of the present disclosure, the UV-induced collagenase expression may be reduced or inhibited, as compared to UV-irradiated cells without treatment with the fermented apple composition.

For example, the fermented apple composition may reduce or inhibit the UV-induced collagenase expression in a range consisting of one lower limit selected from 10% or more, 15% or more, 20% or more, 21% or more, 22% or more, 23% or more, 24% or more, 25% or more, 26% or more, 27% or more, 28% or more, 29% or more, and 29.1% or more, and/or one upper limit selected from 100% or less, 50% or less, 40% or less, and 35% or less, but is not limited thereto.

In the present disclosure, the fermented apple composition of the present disclosure may have an effect of improving the skin.

In the present disclosure, the fermented apple composition of the present disclosure may have an effect of preventing skin aging.

As used herein, the term "skin improvement" refers to improvement of skin conditions by an anti-aging effect on the skin. Alternatively, it may refer to improvement or enhancement of skin health conditions.

The aging may be photoaging. More specifically, the aging may be UV-induced skin aging.

The skin improvement or skin anti-aging may be achieved by, for example, inhibiting degradation of collagen, and/or reducing or inhibiting expression of collagenase, which is as described above.

In the present disclosure, the fermented apple composition of the present disclosure may have an effect of inhibiting UV-induced wrinkle formation.

The inhibiting of wrinkle formation may be achieved by, for example, inhibiting degradation of collagen, and/or reducing or inhibiting expression of collagenase, which is as described above.

In the present disclosure, the fermented apple composition of the present disclosure may have an effect of reducing or inhibiting expression of inflammatory cytokines.

The inflammatory cytokine of the present disclosure may be interleukin-8 (IL-8), IL-1α, IL-12, tumor necrosis factor alpha (TNF-α), or interferon gamma (IFN-y), and any inflammatory cytokine may be included in the scope of the present disclosure, as long as it is known in the art. In one embodiment, the inflammatory cytokine may be IL-8.

When inflammation-induced cells are treated with the fermented apple composition, the inflammation-induced inflammatory cytokine expression may be reduced or inhibited, as compared to inflammation-induced cells without treatment with the fermented apple composition.

For example, the fermented apple composition may reduce or inhibit the inflammation-induced inflammatory cytokine expression in a range consisting of one lower limit selected from 10% or more, 15% or more, 20% or more, 25% or more, 30% or more, 35% or more, 40% or more, 45% or more, 46% or more, 50% or more, 54% or more, 55% or more, 60% or more, 65% or more, and 69% or more, and/or one upper limit selected from 100% or less, 90% or less, 80% or less, and 70% or less, but is not limited thereto.

In the present disclosure, the fermented apple composition of the present disclosure may have an anti-inflammatory effect.

In the present disclosure, the fermented apple composition of the present disclosure may have an effect of preventing or improving enteritis.

As used herein, the term "enteritis" refers to inflammation that occurs in the intestine, and refers to inflammation caused by stimulation of the intestine due to viruses, bacteria, ingestion of toxins, *etc.*

As used herein, the term "preventing" means all of the actions by which enteritis is restrained or retarded by the administration of the composition of the present disclosure.

As used herein, the term "improving" means all of the actions by which symptoms in a subject, who is suspected of having enteritis and has developed enteritis, have taken a turn for the better or been modified favorably by the composition.

The anti-inflammation or the prevention or improvement of enteritis may be achieved by, for example, reducing or inhibiting expression of inflammatory cytokines, which is as described above.

In the present disclosure, the fermented apple composition of the present disclosure may have an effect of improving or enhancing intestinal health.

The improvement or enhancement of intestinal health may be achieved by, for example, reducing or inhibiting expression of inflammatory cytokines, which is as described above.

Another aspect of the present disclosure provides a food including the fermented apple composition of the present disclosure.

The fermented apple composition is as described above.

In the present disclosure, the food of the present disclosure may have an effect of improving or enhancing skin health. The improvement or enhancement of skin health may be achieved by, for example, inhibiting degradation of collagen, and/or reducing or inhibiting expression of collagenase, which is as described in the "skin improvement" of the previous aspect.

In the present disclosure, the food of the present disclosure may have an effect of preventing or improving enteritis. The prevention or improvement of enteritis may be achieved by, for example, reducing or inhibiting expression of inflammatory cytokines, which is as described above.

In the present disclosure, the food of the present disclosure may have an effect of improving or enhancing intestinal health. The improvement or enhancement of intestinal health may be achieved by, for example, reducing or inhibiting expression of inflammatory cytokines, which is as described above.

Still another aspect of the present disclosure provides a health functional food including the fermented apple composition of the present disclosure.

The fermented apple composition is as described above.

In the present disclosure, the health functional food of the present disclosure may have an effect of improving or enhancing skin health. The improvement or enhancement of skin health may be achieved by, for example, inhibiting degradation of collagen, and/or reducing or inhibiting expression of collagenase, which is as described in the "skin improvement" of the previous aspect.

In the present disclosure, the health functional food of the present disclosure may have an effect of preventing or improving enteritis. The prevention or improvement of enteritis may be achieved by, for example, reducing or inhibiting expression of inflammatory cytokines, which is as described in the previous aspect.

In the present disclosure, the health functional food of the present disclosure may have an effect of improving or enhancing intestinal health. The improvement or enhancement of intestinal health may be achieved by, for example, reducing or inhibiting expression of inflammatory cytokines, which is as described in the previous aspect.

The "food" of the present disclosure may include all types of general foods, functional foods, nutritional supplements, health foods, food additives, *etc.*

The above type of food may be prepared into various forms according to common methods known in the art.

The food includes forms such as pills, powders, granules, infusions, tablets, capsules, powders or liquids, *etc.,* and foods to which the composition of the present disclosure may be added may include, for example, various kinds of foods, such as rice, edible grain flour (edible grain powder), grain soup, rice bowl, noodles, rice soup, instant rice, condiment , lunch box rice, dried cooked rice, bread, edible sugar, rice cake, bibimjang, sauces, spices, edible salt, seasoning, seasoning powders, processed, frozen, dried and cooked fruits and vegetables, jellies, jams, compotes, eggs, milk and other dairy products, edible oils and fats, coffee, cocoa and coffee substitutes, tapioca, grain flour and grain preparations, ramen, udon, noodles, noodle soup, cold noodles, porridge, soup, soup dishes, instant foods, frozen foods, instant foods, retort foods, other beverages, chewing gum, teas, vitamin complex, health supplements, *etc.,* but is not limited thereto.

As ingredients that may be included in the food of the present disclosure, various herbal extracts, food auxiliary additives, or natural carbohydrates may be included as additional ingredients, like in general foods. Further, the food auxiliary additives may include food auxiliary additives common in the art, for example, flavoring agents, savory agents, coloring agents, fillers, stabilizers, *etc.*

Examples of the natural carbohydrates include monosaccharides, for example, glucose, fructose, *etc*.; disaccharides, for example, maltose, sucrose, *etc*.; and polysaccharides, for example, common sugars such as dextrins, cyclodextrins, *etc.,* and sugar alcohols such as xylitol, sorbitol, erythritol, *etc.* In addition to those described above, natural flavors (*e.g*., rebaudioside A, glycyrrhizin, *etc*.) and synthetic flavors (saccharin, aspartame, *etc*.) may be advantageously used as flavoring agents. In addition, common food additives used for the purpose of supplementing taste and nutrition, for example, nucleic acids, amino acids, organic acids, *etc.,* may be added.

In addition to those described above, the food of the present disclosure may include various nutrients, vitamins, minerals (electrolytes), flavors such as synthetic flavors and natural flavors, *etc.,* coloring agents and fillers (cheese, chocolate, *etc*.)*,* pectic acid or salts thereof, alginic acid and salts thereof, organic acids, protective colloidal thickeners, pH adjusters, stabilizers, preservatives, glycerin, alcohols, carbonation agents used in carbonated drinks, *etc.* In addition, the food may include fruit flesh for the preparation of natural fruit juices, fruit juice beverages, and vegetable beverages. These components may be used independently or in combination.

The food of the present disclosure may be prepared by a method commonly used in the art, and may be prepared by adding raw materials and ingredients commonly added in the art during the preparation. In addition, the formulation of the food may also be prepared without limitation as long as the formulation is recognized as a food.

Further, when the food of the present disclosure is used as a health functional food, the food of the present disclosure may be prepared in various types of formulations, and unlike general medicines, since the food is used as a raw material, there are advantages of being free from side effects that may occur when taken for a long period of time, and of being highly portable, and therefore, the food of the present disclosure may be ingested as a supplement.

Meanwhile, the food of the present disclosure may also include flavoring agents, savory agents, coloring agents, fillers, stabilizers, and flavors which may also be classified as food additives.

As used herein, the term 'flavor' may be a material added to enhance the flavor of food. The flavor may be a material that allows foods to have excellent seasoning property.

The flavor may be divided according to taste components. In other words, according to the taste, the flavor may be divided into neutral flavor, beef flavor, chicken flavor, pork flavor, kokumi flavor, *etc.*

The 'kokumi flavor' means a flavor which releases the flavor of kokumi, and the 'kokumi' is a word of Japanese origin, and may also be expressed as 'mouthfulness', 'continuity', 'thickness', and 'heartiness' in English, and expressed as 'rich taste', 'thick taste', 'mouth-filling taste', 'dense taste', 'sticky taste', *etc.* in Korean. The 'neutral flavor' refers to a flavor that maximizes 'umami' and minimizes other flavors to produce a mild and clean flavor. For example, oils such as canola oil or grapeseed oil among oils may be acknowledged as having a neutral flavor. The term 'seasoning property' may mean to have a sour, sweet, salty, bitter, or umami taste, but is not limited thereto.

The food of the present disclosure may be a CJ CheilJedang Co., Ltd.'s apple vinegar or fruit fermented cider vinegar product, for example, Petitzel Micho^{®} or Micho^{®} product, but is not limited thereto. The food of the present disclosure may include the fermented apple composition of the present disclosure at a concentration of 1%(v/v) to 50%(v/v), 1%(v/v) to 30%(v/v), 5%(v/v) to 50%(v/v), 5%(v/v) to 30%(v/v), or 10%(v/v) to 30%(v/v) with respect to the total volume of the food, but is not limited thereto.

Still another aspect of the present disclosure provides a cosmetic composition including the fermented apple composition of the present disclosure.

The fermented apple composition is as described above.

In the present disclosure, the cosmetic composition of the present disclosure may have an effect of alleviating or improving skin wrinkles. The alleviation or improvement of wrinkles may be achieved by, for example, inhibiting degradation of collagen, and/or reducing or inhibiting expression of collagenase, which is as described in the "inhibition of wrinkle formation" of the previous aspect.

In the present disclosure, the cosmetic composition of the present disclosure may have an effect of improving the skin or preventing skin aging. The improvement of the skin or the prevention of skin aging may be achieved by, for example, inhibiting degradation of collagen, and/or reducing or inhibiting expression of collagenase, which is as described in the previous aspect.

The "cosmetic composition" of the present disclosure may be prepared into a formulation selected from the group consisting of solutions, ointments for external use, creams, foams, nutrient lotions, softening lotions, packs, softening water, emulsions, makeup bases, essences, soaps, liquid cleansers, bath preparations, sunscreen creams, sun oils, suspensions, emulsions, pastes, gels, lotions, powders, soaps, surfactant-containing cleansers, oils, powder foundations, emulsion foundations, wax foundations, patches, and sprays, but is not limited thereto.

The cosmetic composition may further include one or more cosmetically acceptable carriers which are blended in general skin cosmetics, and as common ingredients, for example, oil, water, surfactants, moisturizers, lower alcohols, thickeners, chelating agents, pigments, preservatives, flavoring agents, *etc.* may be appropriately blended, but are not limited thereto. The cosmetically acceptable carrier included in the cosmetic composition of the present disclosure may be appropriately selected by those skilled in the art according to the formulation of the cosmetic composition.

The cosmetic composition of the present disclosure may include the fermented apple composition of the present disclosure at a concentration of 0.001%(v/v) to 2%(v/v), 0.005%(v/v) to 1.9%(v/v), 0.02%(v/v) to 1.8%(v/v), 0.025%(v/v) to 1.75%(v/v), 0.03%(v/v) to 1.7%(v/v), 0.035%(v/v) to 1.65%(v/v), 0.04%(v/v) to 1.6%(v/v), 0.045%(v/v) to 1.55%(v/v), or 0.05%(v/v) to 1.5%(v/v) with respect to the total volume of the cosmetic composition. In one embodiment, the cosmetic composition may include the fermented apple composition of the present disclosure at a concentration of 0.05%(v/v) to 1.5%(v/v) with respect to the total volume of the cosmetic composition, but is not limited thereto.

Still another aspect of the present disclosure provides a method of reducing or inhibiting UV-induced collagenase expression, the method including the step of administering the fermented apple composition of the present disclosure.

The collagenase may be, but is not limited to, MMP-1, which is as described above.

The fermented apple composition may reduce or inhibit the UV-induced collagenase expression by 10% or more when UV-irradiated cells are treated therewith, as compared to UV-irradiated cells without treatment with the fermented apple composition, but is not limited thereto. This is as described above.

Still another aspect of the present disclosure provides a method of improving the skin, the method including the step of administering the fermented apple composition of the present disclosure.

Still another aspect of the present disclosure provides a method of preventing skin aging, the method including the step of administering the fermented apple composition of the present disclosure.

The aging may be photoaging, but is not limited thereto. This is as described above.

Still another aspect of the present disclosure provides a method of preventing UV-induced wrinkle formation, the method including the step of administering the fermented apple composition of the present disclosure

Still another aspect of the present disclosure provides a method of reducing or inhibiting expression of inflammatory cytokines, the method including the step of administering the fermented apple composition of the present disclosure.

The inflammatory cytokine may be IL-8, but is not limited thereto. This is as described above.

The fermented apple composition may reduce or inhibit the inflammation-induced inflammatory cytokine expression by 10% or more when inflammation-induced cells are treated therewith, as compared to inflammation-induced cells without treatment with the fermented apple composition, but is not limited thereto. This is as described above.

Still another aspect of the present disclosure provides an anti-inflammatory method, the method including the step of administering the fermented apple composition of the present disclosure

Still another aspect of the present disclosure provides a method of preventing or improving enteritis, the method including the step of administering the fermented apple composition of the present disclosure.

As used herein, the term "administering" means introducing the composition of the present disclosure to a subject for the purpose of reducing or inhibiting the UV-induced collagenase expression, or improving the skin, preventing skin aging, or inhibiting UV-induced wrinkle formation, or to a subject, in which inflammatory cytokines are excessively expressed or enteritis is suspected, by any suitable method.

The composition of the present disclosure is not particularly limited but is applicable to any subject, as long as the subject is a subject for the purpose of reducing or inhibiting the UV-induced collagenase expression, or improving the skin, preventing skin aging, or inhibiting UV-induced wrinkle formation, or a subject, in which inflammatory cytokines are excessively expressed or the subject is suspected of having enteritis. For example, the composition may be applied to any subject of human and non-human animals such as monkeys, dogs, cats, rabbits, guinea pigs, rats, mice, cows, sheep, pigs, goats, birds, and fish.

The composition of the present disclosure may be administered in an amount effective for reducing or inhibiting the UV-induced collagenase expression, or administered in an amount effective for improving the skin, administered in an amount effective for preventing skin aging, or administered in an amount effective for inhibiting UV-induced wrinkle formation.

In addition, the composition of the present disclosure may be administered in an amount effective for reducing or inhibiting the expression of inflammatory cytokines, or may be administered in an amount effective for preventing or improving enteritis.

The preferred dose of the composition of the present disclosure may vary depending on the subject's conditions and body weight, severity of the disease, the type of drug, the route of administration, and the duration, but may be appropriately selected by those skilled in the art.

In addition, the composition of the present disclosure may be administered through various oral or parenteral routes as long as it is able to reach a target tissue. For example, it may be administered orally, parenterally, subcutaneously, intraperitoneally, intrapulmonary, intranasally, rectally or intravenously, intramuscularly, subcutaneously, intrauterine or intracerebral injection, and for local treatment, if necessary, by any suitable method including intralesional administration, but the route of administration is not limited thereto.

In one embodiment, the fermented apple composition may be administered in the form of a food composition including the same. In this regard, the administration may be oral administration.

In another embodiment, the fermented apple composition may be administered in the form of a health functional food composition including the same. In this regard, the administration may be oral administration.

Still another aspect of the present disclosure provides a method of reducing or inhibiting UV-induced collagenase expression, the method including the step of applying the fermented apple composition of the present disclosure to the skin.

Still another aspect of the present disclosure provides a method of improving the skin, the method including the step of applying the fermented apple composition of the present disclosure to the skin.

Still another aspect of the present disclosure provides a method of preventing skin aging, the method including the step of applying the fermented apple composition of the present disclosure to the skin.

Still another aspect of the present disclosure provides a method of inhibiting UV-induced wrinkle formation, the method including the step of applying the fermented apple composition of the present disclosure to the skin.

As used herein, the term "applying" means bringing the composition of the present disclosure into contact with a subject's skin by any suitable method, and includes all of the actions by which the corresponding composition is intended to be absorbed into the skin, but is not limited thereto.

In one embodiment, the fermented apple composition may be applied to the skin in the form of a cosmetic composition including the same.

Still another aspect of the present disclosure provides use of the fermented apple composition of the present disclosure in reducing or inhibiting the UV-induced collagenase expression.

The collagenase may be MMP-1, but is not limited thereto. This is as described above.

Still another aspect of the present disclosure provides a use of the fermented apple composition of the present disclosure in improving the skin.

Still another aspect of the present disclosure provides a use of the fermented apple composition of the present disclosure in preventing skin aging.

The aging may be photoaging, but is not limited thereto. This is as described above.

Still another aspect of the present disclosure provides use of the fermented apple composition of the present disclosure in inhibiting UV-induced wrinkle formation.

Still another aspect of the present disclosure provides use of the fermented apple composition of the present disclosure in reducing or inhibiting the expression of inflammatory cytokines.

The inflammatory cytokine may be IL-8, but is not limited thereto. This is as described above.

Still another aspect of the present disclosure provides an anti-inflammatory use of the fermented apple composition of the present disclosure.

Still another aspect of the present disclosure provides a use of the fermented apple composition of the present disclosure in preventing or improving enteritis.

Still another aspect of the present disclosure provides a use of the fermented apple composition of the present disclosure in improving or enhancing intestinal health.

Still another aspect of the present disclosure provides a use of the food including the fermented apple composition of the present disclosure in improving or enhancing skin health.

Still another aspect of the present disclosure provides use of the health functional food including the fermented apple composition of the present disclosure in improving or enhancing skin health.

Still another aspect of the present disclosure provides a use of the cosmetic composition including the fermented apple composition of the present disclosure in alleviating or improving skin wrinkles.

Still another aspect of the present disclosure provides a use of the food including the fermented apple composition of the present disclosure in preventing or improving enteritis.

Still another aspect of the present disclosure provides a use of the food including the fermented apple composition of the present disclosure in improving or enhancing intestinal health.

Still another aspect of the present disclosure provides a use of the health functional food including the fermented apple composition of the present disclosure in preventing or improving enteritis.

Still another aspect of the present disclosure provides a use of the health functional food including the fermented apple composition of the present disclosure in improving or enhancing intestinal health.

The terms used herein are as described in the previous aspects.

### [Mode for Carrying Out the Invention]

Hereinafter, the present disclosure will be described in more detail with reference to exemplary embodiments. However, the following exemplary embodiments are merely preferred embodiments for illustrating the present disclosure, and therefore, are not intended to limit the scope of the present disclosure thereto. Meanwhile, technical details not described in this specification may be sufficiently understood and easily implemented by those skilled in the art or similar art.

### Preparation Example 1: Preparation of fermented apple composition

An apple concentrate was a commercially available product with 100% concentration of apples. An apple concentrate made in China, of which a sugar content was 60 brix or more, based on a refractometer, and an acidity was 6% or less, was diluted with distilled water 4 times (w/w) to prepare the apple concentrate at about 20 brix.

A fermented apple composition was prepared by inoculating an apple concentrate with a *Saccharomyces cerevisiae* strain, followed by fermentation at 40°C for 7 days to produce a primary fermentation product, and then inoculating the primary fermentation product with an *Acetobacter* sp. strain, followed by fermentation at 35°C for 4 days to produce a secondary fermentation product. Thereafter, the secondary fermentation product was allowed to stand at room temperature (approximately 25°C) for 5 days or more to produce a fermented apple composition.

To remove microorganisms from the fermented apple composition and the apple concentrate, filtration was performed using a 0.2 µm filter.

### Example 1: Evaluation of photoaging-inhibitory ability in vitro

The photoaging-inhibitory ability of the fermented apple composition and the apple concentrate was analyzed using HaCaT cells, in which overexpression of a collagenase, matrix metalloproteinases-1 (MMP-1) was induced by UVB irradiation.

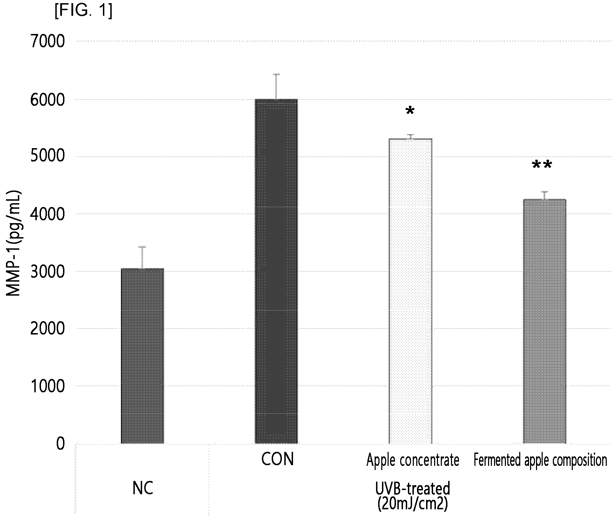

Specifically, the HaCaT cell line (Korea Cell Line Bank) was cultured in an FBS-contained DMEM medium (Gibco, USA) under conditions of 37°C and 5% CO₂ to obtain cells at passages 8 to 12. The obtained HaCaT cells were diluted at a density of 0.7 x 10⁵ cells/well using FBS-contained DMEM, and seeded into a 24-well plate, and cultured for 2 days under conditions of 37°C and 5% CO₂. The medium was replaced by FBS-free DMEM to give the cells starvation conditions, followed by incubation for 1 day (FIG. 1, indicated by NC). Then, the cells were washed twice with Phosphate-Buffered Saline (PBS, Gibco), and then 500 mL of PBS was added to the well, and 20 mJ/cm² of UVB was irradiated to induce MMP-1 overexpression, followed by additional washing with PBS twice (FIG. 1, indicated by CON). As an experimental group, the fermented apple composition and the apple concentrate of Preparation Example 1 were diluted at a concentration of 1%(v/v) with FBS-free DMEM, respectively and then treated to the cells (CON), followed by incubation for 1 day. After culturing, the supernatant was taken and centrifuged at 4°C at 13,000 rpm for 3 minutes to obtain only the supernatant excluding cells. Thereafter, to remove residual bacterial cells in the supernatant, filtration was performed using a 0.2 µm filter.

The concentration of MMP-1 was measured by analyzing the obtained supernatant by an ELISA assay.

In detail, the ELISA assay was performed according to the instructions of the manufacturer of a Human Total MMP-1 DuoSet ELISA (R&D systems) as follows. First, a capture antibody was attached to each well of a 96-well plate overnight. After washing the plate three times with a washing buffer, the resultant was treated with a reagent diluent for 1 hour. Then, after additional washing three times with the washing buffer, the supernatant of each experimental group and control group (NC, CON) and standard solution were added into each well and incubated for 2 hours. After washing them, a detection antibody was added and attached thereto for 2 hours. After additional washing, each well was treated with streptavidin-HRP B for 20 minutes. The reaction was stopped by treating with a reaction stopping solution, and then absorbance was measured at 450 nm, and the MMP-1 concentration was calculated using a 4-parameter logistic.

As a result, both the fermented apple composition and the apple concentrate significantly reduced MMP-1 at concentrations of 1%(v/v), and the fermented apple composition (29.1%) showed the higher MMP-1 reduction rate than the apple concentrate (11.5%) (FIG. 1 and Table 1).

Accordingly, it was confirmed that both the fermented apple composition and the apple concentrate exhibited a photoaging-inhibitory ability, and in particular, the fermented apple composition showed an excellent photoaging-inhibitory ability, as compared to the apple concentrate.

**[Table 1]**

| Treatment group (treatment concentration 1 %(v/v)) | MMP-1 concentration (pg/mL) |
|---|---|
| NC | 3052.0 |
| CON | 6002.6 |
| Apple concentrate | 5311.9 |
| Fermented apple composition | 4256.4 |

### Example 2: Evaluation of anti-inflammatory ability in intestine

The anti-inflammatory ability of the fermented apple composition in the intestine was analyzed by expression levels of human interleukin-8 (hIL-8) in HT-29 cells, in which inflammation was induced by lipopolysaccharide (LPS), according to the method of Lee *et al.* (Lee et al., Biochem Biophys Res Commun 337(2), Nov 2005, pages 457 - 463).

In detail, the HT-29 cell line (Korea Cell Line Bank) was cultured in an RPMI medium (Gibco, USA) supplemented with heat-inactivated 10% fetal bovine serum (FBS), 1% L-glutamine, penicillin G (100 IU/mL) and streptomycin (100 mg/mL) under conditions of 37°C and 5% CO₂. The cultured HT-29 cells were dispensed into a 96-well plate (Corning, USA) at a density of 1.5 x 10⁴ cells/well (volume of 250 µL), and cultured for 3 days until a monolayer was completely formed (FIG. 1, indicated by NC). Then, the supernatant was removed. As an experimental group, each 250 µL/wells of the fermented apple composition (0.25% (v/v), 0.5% (v/v)) and the apple concentrate (0.25% (v/v), 0.5% (v/v)) of Preparation Example 1 was treated together with 0.25 ug/mL of LPS, followed by incubation for 16 hours. The untreated group was treated with only 0.25 µg/mL of LPS, followed by incubation (FIG. 1, indicated by LPS). Although the pH of the fermented apple composition was slightly low, the sample dilution factor was quite high, and the pH did not change due to the buffering effect after dilution with the cell medium. Accordingly, pH adjustment was not performed. After culturing, the supernatant was taken and centrifuged at 4°C at 13,000 rpm for 3 minutes to obtain only the supernatant excluding cells. Thereafter, to remove residual bacterial cells in the supernatant, filtration was performed using a 0.2 µm filter.

The expression levels of hIL-8 were measured by analyzing the obtained supernatant by an ELISA assay.

In detail, the ELISA assay was performed according to the instructions of the manufacturer of a Duoset ELISA ancillary reagent kit 2 (R&D systems, Cat No. DY008, USA) as follows. To perform the ELISA assay, a human IL-8/CXCL8 Quantikine ELISA Kit (R&D systems, Cat No. D8000C, USA) containing an IL-8 antibody and a biotinylated secondary antibody was used. An IL-8 capture antibody (R&D systems, Cat No. 890462) was attached to each well of the ELISA plate at room temperature for 24 hours. The supernatant of each experimental group and control group and IL-8 standard solution (R&D systems, Cat No. 890466) were diluted with a reaction diluent (R&D systems, Cat No. 895877), and 100 µL thereof was added to each well, followed by incubation at room temperature for 2 hours. Thereafter, the culture medium was removed, and a secondary antibody, biotinylated human IL-8 antibody (R&D systems, Cat No. 890465) was added and allowed to attach at room temperature for 2 hours. The color development was induced by treating each well with streptavidin-conjugated horseradish peroxidase (R&D systems, Cat No. 893975) and a substrate thereof, tetramethylbenzidine (TMB, R&D systems, Cat No. DY999). At this time, color development proceeded from blue to yellow. The reaction was stopped by treating with 50 µL of a reaction stopping solution (R&D systems, Cat No. 895032), and then absorbance was measured at 470 nm (correction value at 540 nm) using a spectrophotometer.

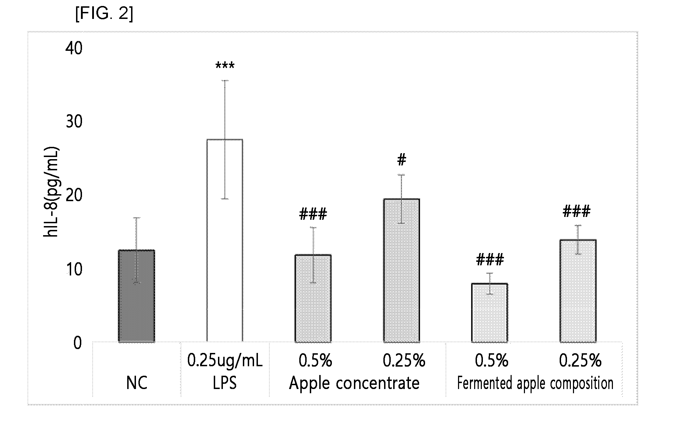

As a result, both the fermented apple composition-treated group and the apple concentrate-treated group showed a significant reduction in the hIL-8 expression level at concentrations of 0.25% (v/v) and 0.5% (v/v) with no cytotoxicity, as compared to the control group (LPS) treated only with LPS (Table 2, FIG. 2, ###p < 0.001, #p < 0.05).

In addition, when comparing the anti-inflammatory ability in the intestine at the same concentrations, the reduction rate of hIL-8 expression level was higher in the fermented apple composition (46% at 0.25%, 69% at 0.5%) than in the apple concentrate (25% at 0.25%, 54% at 0.5%) (Table 2, FIG. 2, ###p < 0.001, #p < 0.05).

Accordingly, it was confirmed that both the fermented apple composition and the apple concentrate exhibited anti-inflammatory effects in the intestine, and in particular, the fermented apple composition had the excellent anti-inflammatory effect, as compared to the apple concentrate.

**[Table 2]**

| **Samples** | **Average** | **St Dev** | **T-test (NO)** | **T-test (LPS)** | | | |
|---|---|---|---|---|---|---|---|
| NC | | 27.6812 5 | 4.355057 2 | 1 | | 4.479E-11 | *** |
| LPS | 0.25ug/ mL | 57.6444 44 | 8.051102 2 | 4.479E-11 | *** | 1 | |
| Apple concentrate | 0.5%(v/v ) | 26.2666 67 | 3.738389 4 | 0.624301 | | 0.0001409 | *** |
| | 0.25%(v/ v) | 43.0666 67 | 3.296799 8 | 3.904E-05 | *** | 0.0192483 | * |
| Fermented apple composition | 0.5%(v/v ) | 17.6666 67 | 1.438363 3 | 0.001667 5 | ** | 1.453E-05 | *** |
| | 0.25%(v/ v) | 30.8 | 1.951068 1 | 0.265348 8 | | 0.0003893 | *** |

### Example 3: Analysis of organic acid and phenolic acid contents

The organic acid and phenolic acid contents of the fermented apple composition and apple concentrate were analyzed.

Specifically, to analyze the organic acid content, the fermented apple composition and apple concentrate of Preparation Example 1 were prepared by diluting each at 1/20 (w/w) in distilled water. The concentrations of six organic acids (citric acid, malic acid, succinic acid, lactic acid, formic acid, and acetic acid) were quantified using LC (Waters, USA), and are shown in Table 3 below.

To analyze the phenolic acid content, the fermented apple composition and apple concentrate of Preparation Example 1 were prepared by diluting each at 1/50(w/w) in 0.1% formic acid. The concentrations of four phenolic acids (gallic acid, chlorogenic acid, caffeic acid, and ferulic acid) were quantified using LC-TQD (Waters, USA), and are shown in Table 4 below.

As a result, it was confirmed that the fermented apple composition had higher contents of succinic acid, lactic acid, formic acid, and acetic acid among organic acids, and higher contents of gallic acid among phenolic acids than the apple concentrate.

**[Table 3]**

| Organic acids (g/L) | Citric acid | Malic acid | Succinic acid | Lactic acid | Formic acid | Acetic acid |
|---|---|---|---|---|---|---|
| Apple concentrate | 0.093 | 4.639 | 0.017 | 0.110 | 0.007 | 0.057 |
| Fermented apple composition | 0.001 g/L or less | 0.153 | **0.412** | **0.826** | **0.023** | **72.419** |

**[Table 4]**

| Phenolic acids (µg/ml (ppm)) | Gallic acid | Chlorogenic acid | Caffeic acid | Ferulic acid |
|---|---|---|---|---|
| Apple concentrate | 0.169 | 56.745 | 3.792 | 0.291 |
| Fermented apple composition | **0.300** | 9.187 | 0.433 | 0.168 |

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the present disclosure is defined by the appended claims rather than by the description preceding them, and therefore all changes and modifications that fall within metes and bounds of the claims, or equivalents of such metes and bounds are therefore intended to be embraced by the claims.

## Claims

1. A fermented apple composition comprising 30 g/L to 100 g/L of acetic acid, and any one or more acids selected from the group consisting of succinic acid, lactic acid, formic acid, and gallic acid.

2. The fermented apple composition of claim 1, wherein the fermented apple composition comprises citric acid in an amount of 0.001 g/L or less.

3. The fermented apple composition of claim 1, wherein the concentration of succinic acid is 0.1 g/L to 0.8 g/L.

4. The fermented apple composition of claim 1, wherein the concentration of lactic acid is 0.2 g/L to 1.5 g/L.

5. The fermented apple composition of claim 1, wherein the concentration of formic acid is 0.01 g/L to 0.05 g/L.

6. The fermented apple composition of claim 1, wherein the concentration of gallic acid is 0.2 g/L to 0.6 g/L.

7. The fermented apple composition of claim 1, wherein the fermented apple composition further comprises any one or more acids selected from the group consisting of malic acid, chlorogenic acid, caffeic acid, and ferulic acid.

8. The fermented apple composition of claim 7, wherein the concentration of malic acid is 0.05 g/L to 0.3 g/L.

9. The fermented apple composition of claim 7, wherein the concentration of chlorogenic acid is 1 g/L to 20 g/L.

10. The fermented apple composition of claim 7, wherein the concentration of caffeic acid is 0.1 g/L to 0.8 g/L.

11. The fermented apple composition of claim 7, wherein the concentration of ferulic acid is 0.05 g/L to 0.3 g/L.

12. The fermented apple composition of claim 1, wherein the fermented apple composition is a composition fermented by inoculating an *Acetobacter* sp. strain into an apple concentrate.

13. The fermented apple composition of claim 12, wherein the fermentation is performed at 20°C to 50°C.

14. The fermented apple composition of claim 12, wherein the fermentation is performed for 2 to 10 days.

15. The fermented apple composition of claim 12, wherein the fermented apple composition is a composition fermented by inoculating a *Saccharomyces cerevisiae* strain into an apple concentrate prior to fermentation by inoculating the *Acetobacter* sp. strain.

16. The fermented apple composition of claim 15, wherein the fermentation is performed at 20°C to 50°C.

17. The fermented apple composition of claim 15, wherein the fermentation is performed for 2 to 10 days.

18. The fermented apple composition of claim 12, wherein the fermented apple composition is a composition fermented with acetic acid by inoculating the *Acetobacter* sp. strain, followed by ripening.

19. The fermented apple composition of claim 18, wherein the ripening is performed at 1°C to 35°C.

20. The fermented apple composition of claim 18, wherein the ripening is performed for 2 to 10 days.

21. The fermented apple composition of claim 1, wherein the fermented apple composition has an effect of reducing or inhibiting UV-induced collagenase expression.

22. The fermented apple composition of claim 21, wherein the collagenase is MMP-1.

23. The fermented apple composition of claim 1, wherein the fermented apple composition has an effect of improving the skin.

24. The fermented apple composition of claim 1, wherein the fermented apple composition has an effect of preventing skin aging.

25. The fermented apple composition of claim 24, wherein the aging is photoaging.

26. The fermented apple composition of claim 1, wherein the fermented apple composition has an effect of inhibiting UV-induced collagen degradation.

27. The fermented apple composition of claim 1, wherein the fermented apple composition reduces or inhibits the UV-induced collagenase expression by 10% or more when UV-irradiated cells are treated therewith, as compared to UV-irradiated cells without treatment with the fermented apple composition.

28. The fermented apple composition of claim 1, wherein the fermented apple composition has an effect of inhibiting UV-induced wrinkle formation.

29. The fermented apple composition of claim 1, wherein the fermented apple composition has an effect of reducing or inhibiting expression of inflammatory cytokines.

30. The fermented apple composition of claim 1, wherein the fermented apple composition reduces or inhibits the inflammation-induced inflammatory cytokine expression by 10% or more when inflammation-induced cells are treated therewith, as compared to inflammation-induced cells without treatment with the fermented apple composition.

31. The fermented apple composition of claim 1, wherein the inflammatory cytokine is IL-8.

32. The fermented apple composition of claim 1, wherein the fermented apple composition has an anti-inflammatory effect.

33. A food comprising the fermented apple composition of claim 1.

34. The food of claim 33, wherein the food is for improving or enhancing skin health.

35. The food of claim 33, wherein the food is for preventing or improving enteritis.

36. The food of claim 33, wherein the food is for improving or enhancing intestinal health.

37. A health functional food comprising the fermented apple composition of claim 1.

38. The health functional food of claim 37, wherein the health functional food is for improving or enhancing skin health.

39. The health functional food of claim 37, wherein the health functional food is for preventing or improving enteritis.

40. The health functional food of claim 37, wherein the health functional food is for improving or enhancing intestinal health.

41. A cosmetic composition comprising the fermented apple composition of claim 1.

42. The cosmetic composition of claim 41, wherein the cosmetic composition is for alleviating or improving skin wrinkles.
